# EUROPEAN PATENT SPECIFICATION

(11) **EP 4 452 269 B1**
(45) Date of publication and mention of the grant of the patent: **03.09.2025**
(21) Application number: 22843140.9
(22) Date of filing: 19.12.2022
(51) Int. Cl.: A61K 31/496, A61K 31/58, A61K 31/7036, A61K 9/00, A61P 31/04, A61P 31/10, A61K 9/10, A61K 47/06

(54) **A PHARMACEUTICAL COMPOSITION FOR THE TREATMENT OF OTIC INFECTIONS**
PHARMAZEUTISCHE ZUSAMMENSETZUNG ZUR BEHANDLUNG VON OHRINFEKTIONEN
COMPOSITION PHARMACEUTIQUE POUR LE TRAITEMENT D'INFECTIONS DE L'OREILLE

(30) Priority: 20.12.2021 US 202163291673 P
(43) Date of publication of application: 30.10.2024
(73) Proprietor: Intervet International B.V., 5831 AN Boxmeer (NL)
(72) Inventor: MARTIN, Sharon Cruz, Rahway, New Jersey 07065 (US); WEINGARTEN, Allan, Madison, New Jersey 07940 (US); FREEHAUF, Keith, Madison, New Jersey 07940 (US); THIRY, Julien, Madison, New Jersey 07940 (US)
(74) Representative: Intervet International B.V.
(86) International application number: PCT/EP2022/086649
(87) International publication number: WO 2023/117900

(56) References cited:
- ANONYMOUS: "Mometamax", MERCK ANIMALL HEALTH, 31 December 2016 (2016-12-31), XP093030282, Retrieved from the Internet <URL:https://www.merck-animal-health-usa.com/channel-content/species/canine/mometamax-otic-suspension> [retrieved on 20230309]
- INTERVET INC: "Compendium of Veterinary Products -Posatex Otic Suspension (MERCK ANIMAL HEALTH) Label Label Use/Dose Use/Dose Posatex Otic Suspension", MERCK ANIMAL HEALTH, 28 February 2019 (2019-02-28), pages 1 - 2, XP093030301, Retrieved from the Internet <URL:https://merckusa.cvpservice.com/product/basic/view/1047303> [retrieved on 20230309]
- ANONYMOUS: "MSD Animal Health Receives Positive CVMP Opinion for MOMETAMAX ULTRA Ear Drops, Suspension for Dogs - Corporate Home Page - MSD Animal Health", MSD ANIMAL HEALTH, 22 October 2022 (2022-10-22), XP093030310, Retrieved from the Internet <URL:https://www.msd-animal-health.com/2022/10/24/msd-animal-health-receives-positive-cvmp-opinion-for-mometamax-ultra-ear-drops-suspension-for-dogs/> [retrieved on 20230309]
- PATERSON S.: "Topical ear treatment - options, indications and limitations of current therapy : Topical ear treatment", JOURNAL OF SMALL ANIMAL PRACTICE, vol. 57, no. 12, 16 October 2016 (2016-10-16), Hoboken, USA, pages 668 - 678, XP093030428, ISSN: 0022-4510, Retrieved from the Internet <URL:https://api.wiley.com/onlinelibrary/tdm/v1/articles/10.1111%2Fjsap.12583> DOI: 10.1111/jsap.12583

## Description

### BACKGROUND

Otitis externa is an inflammatory disease of the external ear canal, including the ear pinna. Otitis externa may be acute or chronic (persistent or recurrent otitis lasting for 3 months or longer) and may involve one or multiple etiologies. The bacteria most commonly isolated from ear canals of dogs affected by otitis are *Staphylococcus* spp. Other bacteria commonly associated with otitis include *Pseudomonas, Proteus, Enterococcus, Streptococcus,* and *Escherichia coli.* Some bacteria such as *Staphylococcus* and *Pseudomonas* may produce biofilm, which can lead to persistence of infection despite adequate therapy, as the biofilm needs to be disrupted for any antimicrobial therapy to be effective in clearing the infection. *Malassezia* yeast is another common component of otitis externa in dogs. Some dogs appear to develop an allergic response to *Malassezia* spp., leading to significant discomfort and pruritus. Effective treatment of ear infection includes treatment of infection and inflammatory changes as well as determination of the underlying factors that led to development of otitis in the first place. Topical therapy is the mainstay treatment for otitis externa although systemic use of anti-inflammatory therapy and/or antimicrobial therapy may be indicated for individual patients. Most dogs with otitis, irrespective of its cause, will benefit from anti-inflammatory therapy. Glucocorticoids can be used for a short duration to help with reduction of pain and swelling, thus helping with improved compliance for ear cleaning and medication administration. Glucocorticoids can also help disrupt biofilm formation and prevent development of chronic otic changes. Long courses or dependence on glucocorticoids for management of otic disease is not encouraged unless it is necessary. Typically, systemic antibiotic therapy as a first line therapy for treatment of otitis externa is discouraged. See J. Bajwa, Can Vet J. 2019 Jan; 60(1): 97-99, the Merck Veterinary Manual, 8th Edition, 1998, pp 372-376 and Aziz et al, Asian Pacific Journal of Tropical Biomedicine,Volume 6, Issue 5, 2016, pp 390-395.

Otomax^{®} (gentamicin sulfate, betamethasone valerate, and clotrimazole ointment) is indicated for the treatment of canine acute and chronic otitis externa associated with yeast (*Malassezia pachydermatis*) and/or bacteria susceptible to gentamicin. Each gram of Otomax^{®} Otic Ointment contains gentamicin sulfate, USP equivalent to 3 mg gentamicin base; betamethasone valerate, USP equivalent to 1 mg betamethasone; and 10 mg clotrimazole, USP in a mineral oil-based system containing a plasticized hydrocarbon gel.. Otomax^{®} is to be administered in two doses per day for 7 days. See NADA 140-896 Otomax^{®}, approved June 9, 1993.

Mometamax^{®} Otic Suspension (gentamicin sulfate, mometasone furoate monohydrate, and clotrimazole, otic suspension) is indicated for the treatment of otitis externa in dogs caused by susceptible strains of yeast *(Malassezia pachydermatis)* and bacteria (*Pseudomonas spp.* [including *P. aeruginosa*]*,* coagulase positive staphylococci, Enterococcus faecalis, Proteus mirabilis, and beta-hemolytic streptococci). Each gram of Mometamax^{®} Otic Suspension contains gentamicin sulfate, equivalent to 3 mg gentamicin base; mometasone furoate monohydrate equivalent to 1 mg mometasone furoate; and 10 mg clotrimazole, in a mineral oil-based system containing a plasticized hydrocarbon gel. Mometamax ^{®} is to be administered in one dose per day for 7 days. See Mometamax ^{®} product insert and NADA 141-177 Freedom of Information Summary, January 9, 2003.

Posatex^{®} Otic Suspension (orbifloxacin, mometasone furoate monohydrate and posaconazole, suspension) is veterinary pharmaceutical product that is used for the treatment of otitis externa in dogs associated with susceptible strains of yeast *(Malassezia pachydermatis)* and bacteria (coagulase positive staphylococci, *Pseudomonas aeruginosa,* and *Enterococcus faecalis*)*.* Each gram of Posatex^{®} Otic Suspension contains 10 mg of orbifloxacin; mometasone furoate monohydrate equivalent to 1 mg mometasone furoate; and 1 mg of posaconazole. Posatex^{®} is to be administered as one dose per day for 7 days. See NADA 141-266 FOI, dated February 18, 2010 and WO 2006/020689.

Neptra^{®} (known as Claro^{®} in the United States) is a veterinary medicine used to treat dogs with short lived or recurrent ear infections (otitis externa) caused by two organisms: Staphylococcus pseudintermedius (a bacterium) and Malassezia pachydermatitis (a yeast). Neptra^{®} contains three active substances: florfenicol, terbinafine and mometasone. One dose (1 ml) Neptra contains: 16.7 mg of florfenicol, 16.7 mg of terbinafine hydrochloride (equivalent to 14.9 mg terbinafine base) and 2.2 mg of Mometasone furoate. Neptra^{®} also contains as excipients propylene carbonate, propylene glycol, ethanol (96 per cent), Macrogol 8000 and purified water. The product is presented in a single-use sealed tube containing 1 ml solution. The recommended dosage of Neptra^{®} is a single-dose container (i.e. 1 ml of solution) per infected ear. Neptra^{®} is considered to have serious eye irritating potential to both animals and people. See EMA/560411/2019, EMEA/V/C/004735,

European Medicines Agency, updated October 2019 and the NOAH Compendium (http://www.noahcompendium.co.uk), accessed September 24, 2021. Neptra^{®} is also called Claro^{®} otic solution. See NADA 141-440 Freedom of Information Summary, September 20, 2015. Osurnia^{®} is a veterinary medicine that contains three active substances: terbinafine, florfenicol and betamethasone acetate. Osurnia^{®} is used to treat short lived or recurrent ear infections (otitis externa) due to Staphylococcus pseudintermedius (a bacterium) and Malassezia pachydermatitis (a yeast). Osurnia^{®} otic gel contains 10 mg of florfenicol, 10 mg of terbinafine and 1 mg of betamethasone acetate (equivalent to 0.9 mg of Betamethasone base) per mL in an off-white to slightly yellow translucent gel. The product contains as excipients butylhydroxytoluene (E 321), Hypromellose, lecithin, oleic acid, propylene carbonate and glycerol formal. Treatment with Osurnia^{®} requires two doses administered seven (7) days apart. See EMA/315814/2014, EMEA/V/C/003753, European Medicines Agency, June 2014.

Neptra^{®} and Osurnia^{®} are used for the treatment of otitis externa associated with susceptible strains of bacteria *(Staphylococcus pseudintermedius)* and yeast *(Malassezia pachydermatis)* in dogs. A disadvantage of these veterinary medicines is that some of their active compounds are not optimal for combatting bacteria or fungi that cause otitis externa.

There is a need of a composition for the treatment of otitis in animals that is safe, has broad antimicrobial efficacy and is administered once to each infected ear.

### SUMMARY OF THE INVENTION

The present invention is defined by the claims. The references to methods of treatment by therapy of this description are to be interpreted as references to compounds, pharmaceutical compositions and medicaments of the present invention for use in those methods.

The present inventors identified a combination of selected active compounds that form a pharmaceutical composition that demonstrates several favorable properties. Therefore, the invention is directed to:
a pharmaceutical composition for treating of an otic infection in an animal comprising
   a) an effective amount of 0.40 to 1.00 % w/v gentamicin or a pharmaceutically acceptable salt thereof;
   b) an effective amount of 0.15 to 0.35 % w/v posaconazole;
   c) an effective amount of 0.15 to 0.35 % w/v mometasone or a pharmaceutically acceptable salt thereof; and
   d) a pharmaceutically acceptable carrier.

The present invention is directed to a pharmaceutical composition comprising gentamicin, posaconazole and mometasone. This composition is useful in treating otic infections in animals, particularly in dogs.

The unique combination of these three active ingredients (Gentamicin, Mometasone Furoate, Posaconazole) at higher concentrations and excipients results in a stable, efficacious product to treat ear infections with a single dose administration.

The claimed pharmaceutical composition provides several advantages over the known medicines used to treat otic infections.

### Advantages

The main advantage of the claimed composition comprising the three active compounds is that it requires only one administration to treat or cure an otic infection in an animal/dog. In addition, the claimed composition can be formulated as a stable suspension with no potential adverse safety concerns upon handling the composition.

The claimed composition can be administered as a single dose treatment when compared to prior otic treatments such as Otomax^{®}, Mometamax^{®}, Posatex^{®} and Osurnia^{®} which require multiple doses to be administered to achieve treatment.

When the claimed composition is administered to the ear of an animal, enough gentamicin remains in the ear canal to prevent spread of gentamicin resistant bacteria.

Gentamicin, the antibiotic used in the claimed composition has broader anti-microbial spectrum than florfenicol (the antibiotic used in Neptra^{®} & Osurnia^{®}). Mometasone Furoate exhibits greater anti-inflammatory potency and a longer duration than betamethasone (the anti-inflammatory component of Osurnia^{®}).

The claimed pharmaceutical composition is a self-preserving formulation and thus does not require a preservative. It remains stable for extended periods of time on the shelf. Based on accelerated aging studies, it has been determined that this pharmaceutical composition will remain stable on the shelf for 36 months. This pharmaceutical composition can also be stored in a multi dose bottle which can be opened and closed multiple times. Once opened by the user, the pharmaceutical composition has been demonstrated to be stable for a period of 3 months. This feature provides convenience to the veterinarian when administering the formulation. The multi-dose container, which can treat up to 20 individual animals, also reduces the amount of packaging waste produced during the treatment of each animal.

The clinical response of the claimed formulation is rapid (within 7 days) and lasts for at least 1 month.

The active pharmaceutical ingredients (API) persist in the ear 45 days after treatment.

Gentamicin, Mometasone Furoate and Posaconazole Otic Suspension is used for the treatment of otitis externa associated with susceptible strains of bacteria sensitive to gentamicin (*Streptococcus* spp., *Staphylococcus pseudintermedius,* beta-hemolytic *Streptococcus canis, Pseudomonas aeruginosa, Escherichia coli, Proteus* spp., *Proteus mirabilis, Enterococcus* spp) and fungi sensitive to posaconazole *(Malassezia pachydermatis)* in dogs. The claimed formulation is effective against otic infections caused be multiple species of bacteria and fungi.

Gentamicin is a broad-spectrum aminoglycoside antibiotic produced by fermentation by *Micromonospora purpurea or M. echinospora.* Gentamicin is an antibiotic complex consisting of four major (C1, C1a, C2, and C2a) and several minor components. This agent irreversibly binds to the bacterial 30S ribosomal subunit. Specifically, this antibiotic is lodged between 16S rRNA and S12 protein within the 30S subunit. This leads to interference with translational initiation complex, misreading of mRNA, thereby hampering protein synthesis and resulting in bactericidal effect. See Pubchem https://pubchem.ncbi.nlm.nih.gov/compound/Gentamicin.

Mometasone is a synthetic steroid hormone in the glucocorticoid family. Glucocorticoid hormones are potent anti-inflammatory agents. It also shows antipruritic and vasoconstrictive actions. It is used topically in the treatment of corticosteroid-responsive dermatoses such as psoriasis and atopic dermatitis. Mometasone Furoate is an anti-inflammatory corticosteroid having the chemical name, 9,21-Dichloro-l 1 (beta), 17-dihydroxy-16 (alpha)-methylpregna-l,4-diene-3,20-dione 17-(2 Furoate). It is practically insoluble in water; slightly soluble in methanol, ethanol, and isopropanol; soluble in acetone and chloroform; and freely soluble in tetrahydro furan. Its partition coefficient between octanol and water is greater than 5000. Mometasone can exist in various hydrated, crystalline, and enantiomeric forms, e.g., as a Monohydrate.

Posaconazole is a broad-spectrum, second generation, triazole compound with antifungal activity. Posaconazole strongly inhibits 14-alpha demethylase, a cytochrome P450-dependent enzyme. Inhibition of 14-alpha-demethylase prevents the conversion of lanosterol to ergosterol, an important component of the fungal cell wall. Inhibition of ergosterol synthesis changes the fungal cell membrane composition and integrity, alters membrane permeability and eventually leads to fungal cell lysis. Compared to other azole antifungals, posaconazole is a significantly more potent inhibitor of sterol 14-alpha demethylase. See Pubchem.

An effective amount of an active ingredient, i.e. gentamicin, posaconazole or mometasone) is the amount of the active ingredient required to treat the otic infection in an ear of the animal in a single dose.

Pharmaceutically acceptable carriers are materials that can transport the active ingredient(s) to the location where needed on or in the patient and are compatible with the other components of the formulation. Examples of pharmaceutically acceptable carriers are mineral oil, isopropyl myristate glycerol, dimethylacetamide, monothioglycerol, polysorbate, propylene glycol, microcrystalline cellulose, sodium carboxymethylcellulose, , triacetin, cellulose acetate, isopropyl myristate, silica, stearyl alcohol, diethylene glycol monoethyl ether, an oil, water or mixtures thereof.

Mineral oil is a clear, odorless liquid and a common ingredient in a variety of cosmetics and personal care products. Mineral oil is made from highly refined, purified and processed petroleum. See US Pharmacopeia Reference 1443952 and CAS # 8012-95-1. The mineral oil is also known as liquid paraffin oil (e.g. CAS number: 8042-47-5, generally available as Drakeol^{®} 6VR (Penreco),

Marcol^{®} 52 (Exxon Mobile), and Klearol^{®} (Sonneborn).

Thickening agents, also known as viscosity enhancing agents can be incorporated into the composition according to the invention and include, but are not limited to, carboxyvinyl polymers, carrageenan, hydroxyethyl cellulose, laponite and water-soluble salts of cellulose ethers such as sodium carboxymethylcellulose and sodium carboxymethyl hydroxyethyl cellulose, methylcellulose, hydroxypropyl-methylcellulose, hydroxypropylcellulose, sodium alginate, carbomer, and povidone. Natural gums such as acacia, gum karaya, xanthan gum, gum arabic, and gum tragacanth can also be used. Other thickening agents include colloidal magnesium aluminum silicate or finely divided silica which can be used as part of the thickening agent to further improve texture. Compositions of the present invention may include 25% to 45% w/v thickening agents.

A preferred thickening agent is Plastibase^{®} 50W( Bristol-Myers Squibb), a plasticized hydrocarbon gel. Plastibase^{®} 50W contains 5% polyethylene in 95% mineral oil. Polyethylene is an inert hydrocarbon with a high molecular weight and high melting point. It is used as a thickening agent to increase the viscosity of the mineral oil and therefore of the drug product. Another preferred thickening agent is Pioneer^{®} PLW plasticized hydrocarbon gel (Hansen & Rosenthal).

Minimum inhibitory concentration (MIC) is defined as the lowest concentration of an antimicrobial that inhibits visible bacterial growth in culture. Therapeutically, this value is used to extrapolate the concentration that an antimicrobial must exceed at the site of the infection to be considered effective. The "susceptible" MIC breakpoint is an in vitro-determined value (in µg/mL) that is applied in clinical microbiology to indicate that a bacterial isolate is susceptible to a given antimicrobial. The susceptible designation is a category that implies that an infection due to that organism may be appropriately treated with the dosage regimen recommended. Susceptibility to an antimicrobial is used by veterinarians to guide appropriate drug selection.

The effective amount of the gentamicin or a pharmaceutically acceptable salt thereof in an amount of about 0.40 to about 1.00 % w/v.

The effective amount of the posaconazole is an amount of about 0.15 to about 0.35 % w/v.

The effective amount of the mometasone or a pharmaceutically acceptable salt thereof is an amount of about 0.15 to about 0.35 % w/v.

In an embodiment of the invention, the pharmaceutical composition further comprises a thickening or viscosity enhancing agent.

In an embodiment of the invention, the thickening agent is a plasticized hydrocarbon gel.

In an embodiment of the invention, the plasticized hydrocarbon gel is 5% polyethylene in 95% mineral oil.

In an embodiment of the invention, the thickening agent is in an amount of about 25% to 40% w/v, about 25% to 45% w/v, about 28% to 35% w/v or about 40%.

In an embodiment of the invention, the pharmaceutical composition is a suspension.

In an embodiment of the invention, the pharmaceutically acceptable carrier is a mineral oil.

In an alternative embodiment, the pharmaceutically acceptable carrier is glycerol, dimethylacetamide, monothioglycerol, or mixtures thereof.

In an alternative embodiment, the pharmaceutically acceptable carrier is glycerol, polysorbate, propylene glycol, microcrystalline cellulose, sodium carboxymethylcellulose, , water or mixtures thereof.

In an alternative embodiment, the pharmaceutically acceptable carrier is triacetin, cellulose acetate, monothioglycerol or mixtures thereof.

In an alternative embodiment, the pharmaceutically acceptable carrier is isopropyl myristate, silica, stearyl alcohol, diethylene glycol monoethyl ether or mixtures thereof.

In an embodiment of the invention, the gentamicin is gentamicin sulfate.

In an embodiment of the invention, the mometasone is mometasone furoate monohydrate.

In an embodiment of the invention, the pharmaceutical composition is stable when stored in a multidose container for an extended period of time.

In the context of this invention, an extended period of time is about 36 months.

In another embodiment of the invention, the pharmaceutical composition is stored in a multidose container.

In an embodiment of the invention, the multidose container contains about 10 doses to about 100 doses, preferably at least 20 doses.

An alternative embodiment of the invention is a method of treating an otic infection in an animal comprising administering to an ear of the animal anyone of the above pharmaceutical compositions.

In an embodiment of the invention, the pharmaceutical composition is administered once.

In an embodiment of the invention, the animal is a dog.

In an embodiment of the invention, the animal is a cat.

In an embodiment of the invention, the otic infection is cause by one or more of *Staphylococcus pseudintermedius,* beta-hemolytic *Streptococcus* spp., *Pseudomonas aeruginosa, Escherichia coli, Proteus* spp., *Enterococcus* spp., or *Malassezia pachydermatis.*

### Examples

### Example 1 - Preparation of the pharmaceutical composition

A 175 L (153 kg) batch of the pharmaceutical formulation was manufactured according to the following process:
1. Charged approximately 80% of the paraffin liquid to the main compounding tank.
2. Charged approximately 8% of the paraffin liquid to a premix vessel.
3. Charged the required amount of gentamicin sulfate to the premix tank from step 2. Mixed with a homogenizer for approximately 5 minutes.
4. Mometasone added
   A) the required amount of mometasone furoate monohydrate was weighed into a 5 L bottle.
   B) 2 kg of mineral oil (paraffin liquid) was added to the bottle and shake until the mometasone furoate monohydrate is well dispersed.
   C) the contents of the bottle was transferred to the premix vessel.
   D) the bottle was rinsed three times with 1 kg of mineral oil (paraffin liquid) and the rinsate was transfered to the premix vessel.
   E) the contents of the premix tank were mixed with a homogenizer for approximately 5 minutes.
5. the required amount of posaconazole was charged to the premix vessel. Mix with a homogenizer for approximately 5 minutes.
6. the premix was charged to the main compounding tank while mixing. The premix tank was rinsed with a part of the paraffin liquid and add the rinses to the main compounding tank.
7. Homogenized the main compounding tank with an in-line, recirculating homogenizer for a minimum of 60 minutes.
8. Continuously charged the required amount of plasticized hydrocarbon gel to the batch while mixing. Continued mixing for approximately 18 minutes. Maintained a product temperature of ≤35°C in the main compounding tank
9. Continued to mix the bulk suspension with a low shear agitator while initiating homogenization with an in-line, recirculating homogenizer for a minimum of 70 minutes.
10. After completion of step 9, continued to mix the bulk suspension during filling with a low shear agitator.
11. Filled the batch into HDPE bottles and closed with a cap.

The resulting formulation is described in Table 1.

**Table 1**

| **Component** | **Function** | **Concentration (mg/mL)** | **% w/v** |
|---|---|---|---|
| Gentamicin (as the sulfate), Micronized | antibiotic | 8.6^{a} | 0.86 |
| Mometasone Furoate (as the monohydrate), Micronized | Anti-inflammatory | 2.1^{b} | 0.21 |
| Posaconazole, Micronized | Anti-fungal | 2.6 | 0.26 |
| Mineral Oil / Paraffin Liquid | Oleaginous Vehicle | 580.0 | 58.0 |
| Plasticized Hydrocarbon Gel | thickening agent | QS to 1.0 mL | QS to 100% |

| | | | |
|---|---|---|---|
| a Gentamicin sulfate added is adjusted based on purity to achieve 8.6 mg of gentamicin base per mL of product. b 2.1 of mometasone furoate is equivalent to 2.2 mg mometasone furoate monohydrate per mL of product | | | |

### Example 2 - Gentamicin Concentration in Pilot Ear Depletion Study

According to minimum inhibitory concentration (MIC) studies for gentamicin performed in the US, the highest MIC with gentamicin resistant *Pseudomonas aeruginosais* is 64 µg/mL. An ear depletion study was performed to assess gentamicin concentration in ear wax. It was determined that the gentamicin concentrations detected in the ear wax were high enough to be effective against gentamicin resistant *Pseudomonas aeruginosa.*

**Table 2**

| Time Point (Hours) Post Dose | Ear (Right or Left) | Total Gentamicin Concentration (mg/g) [Mean ± STD] |
|---|---|---|
| 24 | Left | 11.51 ± 8.68 |
| 24 | Right | 11.96 ±10.11 |
| 72 | Left | 8.15 ± 2.94 |
| 72 | Right | 8.66 ± 5.00 |
| 168 | Left | 3.78 ± 1.55 |
| 168 | Right | 3.97 ± 3.38 |
| 240 | Left | 1.87 ± 1.49 |
| 240 | Right | 2.29 ± 1.36 |
| 336 | Left | 1.46 ± 2.08 |
| 336 | Right | 2.25 ± 2.44 |

### Example 3 Efficacy of the pharmaceutical composition in treatment of otic infection in dogs

The objective of this study was to evaluate the efficacy and safety of a single dose (0.8 mL) of an investigational veterinary product (IVP) which is an otic suspension pharmaceutical composition (prepared as described in Example 1) containing 8.6 mg/mL gentamicin, 2.1 mg/mL mometasone furoate and 2.6 mg/mL posaconazole against bacterial and/or fungal otitis externa in client owned dogs at multiple sites. The efficacy was compared to a registered product (Osurnia^{®}, Elanco Europe Ltd.) (the control product).

### Materials and Methods

This GCP-compliant, multi-center field study was positive - controlled and investigator blinded. It was conducted in 316 client-owned dogs suffering from otitis externa. 153 animals were treated with IVP and a further 163 animals were treated with the Control Product. On average, the animals were 5.7 years old in each treatment group and weighed 24.1 kg (IVP group) and 22.4 kg (CP group). On study day (SD) 0, animals presenting with an ear examination score of at least 5 in at least one ear using the OTIS-3 scoring criteria (see Nuttall and Bensignor, Vet Dermatol 2014; 25: 530) were eligible for inclusion. On SD 0, a physical examination, ear examination (otic scoring), swab sampling and treatment of the inflamed ear(s) were performed. On SD 7, 14 ± 2, 28 ± 2 and 42 ± 2, physical examinations and otic scorings were performed. Animals of the Control group received treatment also on SD 7 according to the manufacturer's instructions. Ear swab samples were taken on SD 28 ± 2. On SD 7 and 14 ± 2 ear swab samples were taken only in case of treatment failures, on SD 42± 2 only in case of relapse. Ear swab samples were also taken at any time point after SD 7 if the otitis worsened.

The primary efficacy criterion was the treatment success rate which was defined as follows: total score of ≤ 4 on SD 14 and of ≤ 3 on SD 28. Dogs that were not a treatment success on SD 28 or that were withdrawn from the study before SD 28 (because requiring an alternative treatment of the otitis), were classified as "treatment failures". Secondary efficacy criteria were intermediate treatment success rate (score of ≤ 4 on SD 14), relapse rate (on SD 42 score of ≥5) and microbiological cure rate on SD28.

### Results

The treatment success rate on SD 28 was 89.5% for dogs treated with gentamicin, mometasone furoate and posaconazole otic suspension. This rate is significantly non-inferior (p<0.0001) when compared to the treatment success rate of 87.2 % for dogs treated with Osurnia^{®}. The intermediate treatment success rate on SD 14 was 93.7 % for dogs treated with gentamicin, mometasone furoate and posaconazole otic suspension. This rate is significantly non-inferior (p<0.0001) when compared to the intermediate treatment success rate of 90.2 % for dogs treated with Osurnia^{®}. The relapse rate on SD 42 of dogs that were a treatment success on SD 28 was 4.7% for dogs treated with gentamicin, mometasone furoate and posaconazole otic suspension. This rate is significantly noninferior (p=0.0002) when compared to the relapse rate of 1.7% for dogs treated with Osurnia^{®}. The microbiological cure rate on SD 28 was 81.1 % for *Malassezia pachydermatis* and 73.5% for *Staphylococcus pseudintermedius* in dogs treated with gentamicin, mometasone furoate and posaconazole otic suspension. In dogs treated with Osurnia^{®} twice at an interval of 7 days the microbiological cure rate on SD 28 for *Malassezia pachydermatis* was 83.8% and 90% for *Staphylococcus pseudintermedius.* The microbiological cure rates against *P. aeruginosa, E. coli, S. canis* and *P*. *mirabilis* ranged between 60% and 100% in the IVP group and between 42.9% and 100% in the CP group.

### Conclusion

The new otic suspension containing 8.6 mg/mL gentamicin, 2.1 mg/mL mometasone furoate and 2.6 mg/mL posaconazole administered as a single dose of 0.8 mL was efficacious against canine otitis externa and well tolerated and safe in the tested dose. It has shown significant non-inferiority with regards to treatment success on SD 28, intermediate treatment success on SD 14 and overall treatment success on SD 42 when compared to two treatments with Osurnia^{®}, on SD 0 and SD 7.

These results for the IVP pharmaceutical composition with gentamicin, mometasone and posaconazole were achieved with only one dose while results for the control product (Osurnia^{®}) required two doses, 7 days apart.

### Example 3 - Stability

The pharmaceutical composition of Example 1 (see Table 1 above) was evaluated for long term stability under accelerated conditions. The composition was packaged in 35 mL white HDPE bottles with LDPE caps. The bottles were filled with no less than 24 mL so that at least 20 doses of 0.8 mL each could be taken from each bottle. Removal of each dose was performed with a 1.0 mL syringe, using a LDPE press-in-bottle adapter (PIBA).

The bottles were maintained for 12 months under accelerated conditions (40 °C/75% relative humidity (RH)), refrigerated conditions (5°C/Ambient humidity) and long-term storage conditions (30°C/65% RH and 30°C/75% RH).

Sample doses were taken from each bottle at the time points of 0, 6 and 12 months. Samples were analyzed for appearance, density, viscosity, water content, particle size distribution, weight change and identification and assay of gentamicin, mometasone furoate, and posaconazole. Samples were also evaluated for morphology of the api particles (i.e are the particles still crystals) and agglomeration of the api particles.

The sample dose's appearance was initially described as smooth, uniform white to off-white viscous suspension. Under the stability testing, each of the samples maintained this appearance through the length of the test under each of the conditions applied.

The initial density values of the samples of 0.850 g/mL and 0.900 g/mL (measured at 20°C in accordance with Ph. Eur. 2.2.5) were maintained throughout the stability study at all conditions.

Viscosity (determined on a Brookfield Viscometer) were initially determined to be between 800 and 2200 centipoise. The viscosity was maintained in this range at all conditions throughout the stability testing.

Water content was evaluated by Karl Fischer method and initially determined to be less than 1.0%. There was a slight increase in water content, from 0.1% to 0.4% during accelerated and long-term conditions. The data indicates that the composition does not
pick up sufficient water to impact the efficacy and safety of the composition over the shelf life.

Particle size was measured by laser diffraction. Particle size distribution measured at D₁₀, D₅₀, and D₉₀ was consistent through 12 months at accelerated, refrigerated and long-term conditions. The data indicates that the particle size distribution at D₅₀ is stable throughout the testing period under refrigerated, long term and accelerated conditions.

The optical microscopic data measure if the particles are crystalline (mostly ≤ 10 µm) and the number of agglomerates (i.e., particles >250 µm). For all samples, the crystallinity criteria were met, and zero agglomerates were identified through 12 months at accelerated, refrigerated and long-term conditions. These data indicate that agglomerates do not form even under accelerated conditions.

Weight change varied by < 0.5% through 12 months at accelerated, refrigerated and long-term conditions. The lack of weight change indicates that the container closure system is adequate as packaging material.

The identification and degradation of posaconazole and mometasone furoate was conducted by ultra high pressure liquid chromatography coupled to a UV detector UPLC/UV). The degradation product of posaconazole that was tracked is the formate ester. For mometasone furoate the 17-alcohol and Compound E were tracked. No specified nor unspecified degradation products for mometasone furoate and posaconazole were observed through 12 months at accelerated, refrigerated and long-term conditions.

Gentamicin identification and assay were determined by microbiological titration. Gentamicin assay was maintained at 90%-105% through 12 months at accelerated, refrigerated and long-term conditions.

The available data indicates that the composition does not exhibit any significant changes in regard to critical quality attributes; no special storage condition is required.

### Example 4 In-Use Stability Study

An in-use stability study was conducted to evaluate the stability of the formulation of Example 1 under simulated, in-use conditions for up to 3 months. The sample bottles contained an initial fill volume of 24 mL. In order to dose the correct amount of product, a press-in bottle adapter (PIBA) is inserted into the neck of the bottles to allow for product dosing via a syringe. After each bottle was shaken, 0.8 mL of product was removed in ten aliquots (total = 8 mL) using a syringe. The bottles were then stored at 30°C/65% RH and tested at 3 months. Results of the initial and 3-month samples are summarized in Table 2.

| Table 2 In-Use Stability Results of OTIC Formulation of Example 1 | | | | |
|---|---|---|---|---|
| Test | | Acceptance Criteria^{a} | Initial | 3 months |
| Appearance | | A smooth, uniform, white to off-whiteviscous suspension | A smooth, uniform, off-whiteviscous suspension | A smooth, uniform, off-white viscous suspension |
| Density at 20°C (g/mL) | | 0.825 to 0.925 | 0.868 | 0.877 |
| Viscosity (cP) | | 800 to 3000 | 1622 | 1448 |
| Water Content (%) | | Report to nearest 0.1 | 0.1 | 0.2 |
| Assay of Posaconazole (% LC) | | 90.0 to 110.0 | 98.6 | 97.3 |
| Degradation Products of Posaconazole (%) | Posaconazole | ≤ 1.0 | ND | ND |
| | Formate Ester | ≤ 1.0 | | |
| | Unspecified (each) | ≤ 1.0 | | |
| | Total | ≤ 2.0 | | |
| Assay of Mometasone Furoate (% LC) | | 90.0 to 110.0 | 100.2 | 101.3 |
| Degradation Products of Mometasone Furoate (%) | 17-Alcohol | ≤ 1.0 | ND | ND |
| | Compound E | ≤ 0.6 | | |
| | Unspecified (each) | ≤ 1.0 | | |
| | Total | ≤ 2.5 | | |
| Assay of Gentamicin (% LC) | | 90.0 to 130.0 | 96.2 | 106.8 |
| Packaging Observations | | A white, intact plastic container with a screw cap affixed. No leakage observed,label clearly legible | A white, tightly screwed capped bottle with no signs ofleakage | White, plastic container witha screw cap and no signs of leakage. PIBA insert presentwhen cap unscrewed. |
| Particle Size and Crystal Morphology | Stage 1 (3 slides) | Confirm the presence of crystalline particles. Confirm if the majority of particles are ≤ 10 µm. Report if not morethan 6 agglomerates are > 250 µm. | Typical particle size of 5-10 µm. Particles are crystalline. 0 agglomerates > 250 µm in 3slides. | Typical particle size of 5-10µm. Particles are crystalline. 0 agglomerates > 250 µm in 3 slides. |
| | Stage 2 (Additional 6 slides) | Report the number of agglomerates thatare > 250 µm. | NA | NA |
| Microbiological Quality | Total Aerobic MicrobialCount - TAMC (CFU/g)) | ≤ 10² | < 100 | < 100 |
| | Total Combined Yeast/Molds Count -TYMC (CFU/g) | ≤ 10¹ | < 10 | < 10 |
| | Absence of Pseudomonas aeruginosa | Absent in 1 g | Absence | Absence |
| | Absence of Staphylococcus aereus | Absent in 1 g | Absence | Absence |
| ^{a} Specifications at the time of development, ND: <0.3% (reporting threshold) | | | | |

The in-use stability study was also performed on aged samples of the formulation of Example 1, i.e. at the end of the proposed shelf-life of 24 months. Sample bottles from this batch followed the same in-use procedure as that for previous batch. After each bottle was shaken, 0.8 mL of product was removed in ten aliquots (total = 8 mL) using a syringe. The bottles were then stored at 30°C/65% RH for 24 months and tested up to 3 months after the proposed shelf-life of 24 months. Results are summarized in Table 3.

| Table 3 Aged In-Use Stability Results of OTIC Formulation of Example 1 | | | | |
|---|---|---|---|---|
| Test | Acceptance Criteria¹ | 24-Month Sample | Aged In-Use Sample(24 + 3 months) | |
| Appearance | | A smooth, uniform, white to off-whiteviscous suspension | A smooth, uniform, whiteviscous suspension | A smooth, uniform, whiteviscous suspension |
| Density at 20°C (g/mL) | | 0.825 to 0.925 | 0.879 | 0.878 |
| Viscosity (cP) | | 800 to 3000 | 1416 | 1436 |
| Water Content (%) | | Report to nearest 0. 1 | 0.3 | 0.3 |
| Assay of Posaconazole (% LC) | | 90.0 to 110.0 | 101.4 | 101.1 |
| Degradation Products of Posaconazole (%) | Posaconazole | ≤ 1.0 | ND | ND |
| | Formate Ester | ≤ 1.0 | ND | ND |
| | Unspecified (each) | ≤ 1.0 | ND | ND |
| | Total | ≤ 2.0 | ND | ND |
| Assay of Mometasone Furoate (% LC) | | 90.0 to 110.0 | 101.5 | 101.9 |
| Degradation Products of Mometasone Furoate (%) | 17-Alcohol | ≤ 1.0 | ND | ND |
| | Compound E | ≤ 0.6 | | |
| | Unspecified (each) | ≤ 1.0 | | |
| | Total | ≤ 2.5 | | |
| Assay of Gentamicin (% LC) | | 90.0 to 130.0 | 96.4 | 92.5 |
| Packaging Observations | | A white, intact plastic container with a screw cap affixed. No leakage observed,label clearly legible | Intact plastic white containerwith a screw cap tightly affixed, no leakage observed and label clearly visible. | Intact white screw top bottlewith a legible label securelyaffixed to the bottle. PIBA insert present when cap unscrewed. |
| Particle Size and Crystal Morphology | Stage 1 (3 slides) | Confirm the presence of crystalline particles. Confirm if the majority of particles are ≤ 10 µm. Report if not morethan 6 agglomerates are > 250 µm. | Typical particle size of 5-15µm. Particles are crystalline. 0 agglomerates > 250 µm in 3 slides. | Typical particle size of 5-10µm. Particles are crystalline. 0 agglomerates > 250 µm in 3 slides. |
| | Stage 2 (Additional 6 slides) | Report the number of agglomerates that are > 250 µm. | NA | NA |
| Microbiological Quality | Total Aerobic Microbial Count - TAMC (CFU/g) | ≤ 10² | <100 | <100 |
| | Total Combined Yeast/Mol ds Count - TYMC (CFU/g) | ≤ 10¹ | <10 | <10 |
| | Absence of Pseudomonas aeruginosa | Absent in 1 g | Absence | Absence |
| | Absence of Staphylococcu s aereus | Absent in 1 g | Absence | Absence |
| ¹ Specifications at the time of development; ND: <0.3% (reporting threshold) | | | | |

Although a low gentamicin assay value was observed for the aged samples (92.5% LC), it was still within the expected shelf-life range for this product. The studies demonstrated that Gentamicin, Mometasone Furoate, and Posaconazole Otic Suspension is stable for up to 3 months after first use and at the end of shelf-life, when the product is stored in an HDPE bottle at the proposed long term storage condition (30°C/65% RH).

A shelf life of 36 months is anticipated based upon the accelerated stability data.

## Claims

1. A pharmaceutical composition for treating of an otic infection in an animal comprising
a) an effective amount of 0.40 to 1.00 % w/v gentamicin or a pharmaceutically acceptable salt thereof;
b) an effective amount of 0.15 to 0.35 % w/v posaconazole;
c) an effective amount of 0.15 to 0.35 % w/v mometasone or a pharmaceutically acceptable salt thereof; and
d) a pharmaceutically acceptable carrier.

2. The pharmaceutical composition of claims1, further comprising a thickening agent.

3. The pharmaceutical composition of any one of claims 1-2, wherein the composition is a suspension.

4. The pharmaceutical composition of any one of claims 1-3, wherein the pharmaceutically acceptable carrier is a mineral oil.

5. The pharmaceutical composition of claim 2, wherein the thickening agent is 5% polyethylene in 95% mineral oil.

6. The pharmaceutical composition of claims 2 or 5, wherein the thickening agent is in an amount of 25% to 40% w/v.

7. The pharmaceutical composition according to any one of claims 1 to 6 for use in treating an otic infection by administering it to an ear of the animal.

8. The composition for use of claim 7, wherein the composition is administered once.

9. The composition for use of any one of claims 7-8, wherein the animal is a dog.

10. The composition for use of any one of claims 7-8, wherein the animal is a cat.

11. The composition for use of any one of claims 7-10, wherein the otic infection is cause by one or more of *Staphylococcus pseudintermedius* beta-hemolytic, *Streptococcus canis, Streptococcus* spp., *Pseudomonas aeruginosa, Escherichia coli, Proteus mirabilis Proteus* spp., *Enterococcus* spp. or *Malassezia pachydermatis.*

12. A multidose container which contains the pharmaceutical composition of any one of claims 1-6.

## Patentansprüche

1. Pharmazeutische Zusammensetzung zum Behandeln einer Ohrinfektion bei einem Tier, umfassend
a) eine wirksame Menge von 0,40 bis 1,00 % w/v Gentamicin oder einem pharmazeutisch unbedenklichen Salz davon;
b) eine wirksame Menge von 0,15 bis 0,35 % w/v Posaconazol;
c) eine wirksame Menge von 0,15 bis 0,35 % w/v Mometason oder einem pharmazeutisch unbedenklichen Salz davon; und
d) einen pharmazeutisch unbedenklichen Träger.

2. Pharmazeutische Zusammensetzung nach Anspruch 1, ferner umfassend ein Verdickungsmittel.

3. Pharmazeutische Zusammensetzung nach einem der Ansprüche 1-2, wobei es sich bei der Zusammensetzung um eine Suspension handelt.

4. Pharmazeutische Zusammensetzung nach einem der Ansprüche 1-3, wobei es sich bei dem pharmazeutisch unbedenklichen Träger um ein Mineralöl handelt.

5. Pharmazeutische Zusammensetzung nach Anspruch 2, wobei es sich bei dem Verdickungsmittel um 5 % Polyethylen in 95 % Mineralöl handelt.

6. Pharmazeutische Zusammensetzung nach Anspruch 2 oder 5, wobei das Verdickungsmittel in einer Menge von 25 % bis 40 % w/v vorliegt.

7. Pharmazeutische Zusammensetzung gemäß einem der Ansprüche 1 bis 6 zur Verwendung beim Behandeln einer Ohrinfektion, indem sie an ein Ohr des Tieres verabreicht wird.

8. Zusammensetzung zur Verwendung nach Anspruch 7, wobei die Zusammensetzung einmal verabreicht wird.

9. Zusammensetzung zur Verwendung nach einem der Ansprüche 7-8, wobei es sich bei dem Tier um einen Hund handelt.

10. Zusammensetzung zur Verwendung nach einem der Ansprüche 7-8, wobei es sich bei dem Tier um eine Katze handelt.

11. Zusammensetzung zur Verwendung nach einem der Ansprüche 7-10, wobei die Ohrinfektion durch eines oder mehrere aus beta-hämolytischem *Staphylococcus pseudintermedius, Streptococcus canis, Streptococcus* spp., *Pseudomonas aeruginosa, Escherichia coli, Proteus mirabilis Proteus* spp., *Enterococcus* spp. oder *Malassezia pachydermatis* verursacht wird.

12. Mehrdosenbehältnis, das die pharmazeutische Zusammensetzung nach einem der Ansprüche 1-6 enthält.

## Revendications

1. Composition pharmaceutique pour le traitement d'une infection otique chez un animal comprenant
a) une quantité efficace de 0,40 à 1,00 % p/v de gentamicine ou d'un sel pharmaceutiquement acceptable de celle-ci ;
b) une quantité efficace de 0,15 à 0,35 % p/v de posaconazole ;
c) une quantité efficace de 0,15 à 0,35 % p/v de mométasone ou d'un sel pharmaceutiquement acceptable de celle-ci ; et
d) un support pharmaceutiquement acceptable.

2. Composition pharmaceutique selon la revendication 1, comprenant en outre un agent épaississant.

3. Composition pharmaceutique selon l'une quelconque des revendications 1 et 2, dans laquelle la composition est une suspension.

4. Composition pharmaceutique selon l'une quelconque des revendications 1 à 3, dans laquelle le support pharmaceutiquement acceptable est une huile minérale.

5. Composition pharmaceutique selon la revendication 2, dans laquelle l'agent épaississant est 5 % de polyéthylène dans 95 % d'huile minérale.

6. Composition pharmaceutique selon la revendication 2 ou 5, dans laquelle l'agent épaississant est présent en une quantité de 25 % à 40 % en p/v.

7. Composition pharmaceutique selon l'une quelconque des revendications 1 à 6, destinée à être utilisée dans le traitement d'une infection otique par administration de celle-ci à une oreille de l'animal.

8. Composition pour utilisation selon la revendication 7, dans laquelle la composition est administrée une fois.

9. Composition pour utilisation selon l'une quelconque des revendications 7 à 8, dans laquelle l'animal est un chien.

10. Composition pour utilisation selon l'une quelconque des revendications 7 à 8, dans laquelle l'animal est un chat.

11. Composition pour utilisation selon l'une quelconque des revendications 7 à 10, dans laquelle l'infection otique est provoquée par un ou plusieurs parmi *Staphylococcus pseudintermedius* bêta-hémolytique, *Streptococcus canis, Streptococcus* spp., *Pseudomonas aeruginosa, Escherichia coli, Proteus mirabilis Proteus* spp., *Enterococcus* spp. ou *Malassezia pachydermatis.*

12. Récipient multidose qui contient la composition pharmaceutique selon l'une quelconque des revendications 1 à 6.
